(19) Europäisches Patentamt
European Patent Office
Office européen des brevets

(11) EP 1 757 592 A1

(12) EUROPEAN PATENT APPLICATION
published in accordance with Art. 158(3) EPC

(43) Date of publication:
**28.02.2007 Bulletin 2007/09**

(21) Application number: **05748792.8**

(22) Date of filing: **08.06.2005**

(51) Int Cl.:
***C07D 237/26*** (2006.01) ***A61K 31/502*** (2006.01)
***A61K 31/5377*** (2006.01) ***A61P 35/00*** (2006.01)
***C07D 401/12*** (2006.01)

(86) International application number:
**PCT/JP2005/010494**

(87) International publication number:
**WO 2005/121105 (22.12.2005 Gazette 2005/51)**

(84) Designated Contracting States:
**AT BE BG CH CY CZ DE DK EE ES FI FR GB GR HU IE IS IT LI LT LU MC NL PL PT RO SE SI SK TR**

(30) Priority: **09.06.2004 JP 2004171426**

(71) Applicant: **NIPPON KAYAKU KABUSHIKI KAISHA**
**Tokyo 102-8172 (JP)**

(72) Inventors:
- **SATO, Yoshitaka**
  **3420041 (JP)**
- **SUZUKI, Yoshikazu**
  **1140014 (JP)**
- **YAMAMOTO, Keiichiro**
  **1150042 (JP)**
- **KUROIWA, Shunsuke**
  **3600816 (JP)**
- **MARUYAMA, Sakiko**
  **1150042 (JP)**

(74) Representative: **Gille Hrabal Struck Neidlein Prop Roos**
**Brucknerstrasse 20**
**40593 Düsseldorf (DE)**

# (54) NOVEL 3-PHENYLTETRAHYDROCINNOLIN-5-OL DERIVATIVE AND MEDICINAL USE THEREOF

(57) A 3-phenyltetrahydrocinnolin-5-ol derivative represented by the following general formula (1) (wherein Z represents 2-carboxyethyl, 3-dimethylaminopropyl etc.; X represents trifluoromethyl etc.; X' represents hydrogen, etc.; and Y anti Y' each represents methyl, hydrogen, etc.), a physiologically acceptable salt thereof, or a prodrug of either. They have cytostatic activity against tumor cells and hence are effective as an antitumor agent.

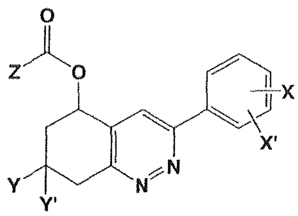

(1)

EP 1 757 592 A1

**Description**

TECHNICAL FIELD

The present invention relates to a 3-phenyltetrahydrocinnolin-5-ol derivative, a physiologically acceptable salt thereof, or a prodrug of either and their medicinal use.

BACKGROUND ART

A malignant tumor is a cluster of cells that continues to proliferate in a living body outside the control of the normal biomechanism and causes the death of the host when not treated. The malignant tumor is generally treated by surgical excision, radiation irradiation, hormonotherapy or chemotherapy. Particularly in the treatment of a malignant solid tumor, a surgical operation is first selected. In general, the radiotherapy, hormonotherapy and chemotherapy are employed as an auxiliary therapy before or after an operation or for treating a malignant solid tumor that is judged to be non-treatable by an operation. The hormonotherapy, chemotherapy and the like are employed for narrowing a region to be excised by an operation and causing the reduction and disappearance of a tumor not completely excisable by the operation to prevent the recurrence of the tumor. The operation, however, causes physical and mental pains to a cancer patient. Moreover, when the tumor has metastasized, a wide region has to be excised and such a manipulation is very difficult at present. The reason why the chemotherapy is not a main method for treating a malignant solid tumor is that in fact, there has been no clinically effective antitumor agent having no serious side effect. Therefore, an antitumor agent having an excellent antitumor effect on a malignant solid tumor is desired.

On the other hand, the following non-patent document 1 describes cinnoline derivatives capable of acting on central nervous system, the non-patent document 2 cinnoline derivatives having inhibitory effect on monoamine oxidase, and the non-patent document 3 the synthesis and reactions of cinnoline-related substances. These references, however, neither describe the 3-phenyltetrahydrocinnolin-5-ol derivatives represented by the general formula (1) shown hereinafler of the present invention nor describe that the 3-phenyltetrahydrocinnolin-5-ol derivative have antitumor effect.

Non-patent document 1:

Rashmi K.Shah et al., Central Nervous System Active 5-Oxo-1, 4, 5, 6, 7, 8-Hexahydrocinnolines, Journal of Medicinal Chemistry, 1976, vol. 19, p.508-511

Non-patent document 2: Angelo Carotti et al., Inhibition of Monoamine Oxidase-B by Condensed Pyridazines and Pyrimidines: Effects of Lipophilicity and Structure-Activity Relationships, Journal of Medical Chemistry, 1998, vol. 41, p. 3812-3820

Non-patent document 3: K.Nagarajan et al., Synthesis, Reactions of 4, 6, 7, 8-Tetrahydro-5-(1H) - cinnolinones, Indian Journal of Chemistry, 1986, vol.25B, p.697-708

DISCLOSURE OF THE INVENTION

Problem to be solved by the Invention

As described above, the reason why the chemotherapy is not a main method for treating a malignant solid tumor is that there has been no effective antitumor agent having a wide anticancer spectrum for malignant solid tumor and no serious side effect. Therefore, an antitumor agent having an excellent antitumor effect on a malignant solid tumor is desired.

Means for Solving the Problem

The present inventors earnestly investigated in order to solve the above problem, and consequently found that a novel 3-phenyltetrahydrocinnolin-5-ol derivative, a physiologically acceptable salt thereof, or a prodrug of either has cytostatic activity and antitumor activity, whereby the present invention has been accomplished.

That is, the present invention relates to a 3-phenyltetrahydrocinnolin-5-ol derivative represented by the following general formula (1):

[Formula 1]

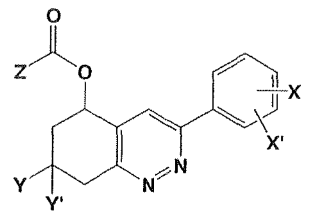

(1)

wherein Z is MO- (O is an oxygen atom), L(L')N- (N is a nitrogen atom) or A(B)CH- (C is a carbon atom and H is a hydrogen atom); M is a lower alkyl group which may have a lower alkoxy group, a lower alkylamino group or a saturated heterocyclic group as a substituent; L and L' may be taken together to represent an optionally substituted 4- to 8-membered cyclic structure group or are independently a lower alkyl group which may have a hydroxyl group, a lower alkoxy group, a carboxyl group, a lower alkoxycarbonyl group, a carbamoyl group, a lower alkylamino group or a saturated heterocyclic group as a substituent, or a hydrogen atom; A is a hydroxyl group, a lower alkyl group or a hydrogen atom; B is a lower alkyl group, a lower alkoxy group, a carboxyl group or a lower alkoxycarbonyl group, which have a substituent(s); X is a lower alkyl group, a lower alkoxycarbonyl group, a lower acylamino group, a lower alkoxy group, a trifluoromethyl group, a nitro group, a cyano group or a halogen atom; X' is a lower alkyl group, a lower alkoxycarbonyl group, a lower acylamino group, a lower alkoxy group, a trifluoromethyl group, a nitro group, a cyano group, a halogen atom or a hydrogen atom; and Y and Y' are independently a lower alkyl group or a hydrogen atom, a physiologically acceptable salt thereof, or a prodrug of said derivative or salt.
In addition, the present invention relates to a pharmaceutical composition comprising the above-mentioned 3-phenyltetrahydrocinnolin-5-ol derivative, a physiologically acceptable salt thereof or a prodrug of said derivative or salt as an active ingredient.
Further, the present invention relates to use of the above-mentioned 3-phenyltetrahydrocinnolin-5-ol derivative, a physiologically acceptable salt thereof or a prodrug of said derivative or salt in the manufacture of a pharmaceutical composition for inhibiting cell proliferation.
Still further, the present invention relates to use of the above-mentioned 3-phenyltetrahydrocinnolin-5-ol derivative, a physiologically acceptable salt thereof or a prodrug of said derivative or salt in the manufacture of a pharmaceutical composition for the prophylaxis or treatment of tumors.
Still further, the present invention relates to a method for inhibiting cell proliferation which comprises administering the above-mentioned 3-phenyltetrahydro-cinnolin-5-ol derivative, a physiologically acceptable salt thereof or a prodrug of said derivative or salt to a mammal including a human being.
Still further, the present invention relates to a method for preventing or treating tumors which comprises administering the above-mentioned 3-phenyltetrahydro-cinnolin-5-ol derivative, a physiologically acceptable salt thereof or a prodrug of said derivative or salt to a mammal including a human being.

Advantages of the Invention

By the present invention, there are provided a novel 3-phenyltetrahydrocinnolin-5-ol derivative represented by the above general formula (1), a physiologically acceptable salt thereof or a prodrug of either, which are effectively usable for preventing or treating tumors; and a pharmaceutical composition effective in the inhibition of cell proliferation and the prophylaxis or treatment of tumors.

BEST MODE FOR CARRYING OUT THE INVENTION

As the lower alkyl group in the present invention, linear or branched (C1~C5)alkyl groups are exemplified. Specific examples thereof are methyl group, ethyl group, n-propyl group, isopropyl group, n-butyl group, sec-butyl group, tert-butyl group, n-pentyl group, isopentyl group, neopentyl group, etc. Of these, preferable examples thereof are methyl group, ethyl group, n-propyl group and isopropyl group.
As the lower alkoxy group in the present invention, linear or branched (C1~C5)alkoxy groups are exemplified. Specific examples thereof are methoxy group, ethoxy group, n-propoxy group, isopropoxy group, n-butoxy group, isobutoxy group, tert-butoxy group, n-pentyloxy group, isopentyloxy group, etc. Of these, preferable examples thereof are methoxy group and ethoxy group.

As the lower alkoxycarbonyl group in the present invention, groups formed by bonding of any of the above-exemplified lower alkoxy group to a carbonyl group are exemplified. The lower alkoxycarbonyl group is preferably a methoxycarbonyl group or an ethoxycarbonyl group.

The lower alkylamino group in the present invention is an amino group having one or two of the above-exemplified lower alkyl groups bonded thereto, and includes, for example, methylamino group, ethylamino group, propylamino group, isopropylamino group, dimethyl amino group and diethylamino group. Of these, dimethylamino group and diethylamino group are preferable.

As the saturated heterocyclic group in the present invention, there are exemplified saturated 5-to 7-membered ring groups which contain 1 to 3 heteroatoms selected from N, O and S and may be substituted by a (C1~C5)alkyl group. The saturated heterocyclic group includes, for example, pyrrolidino group, 4-methylpiperazino group, morpholino group and piperidino group.

When L and L' are taken together to represent an optionally substituted 4-to 8-membered cyclic structure group in L(L')N-, i.e., a group as which Z is defined, said optionally substituted group includes, for example, azetidino group, pyrrolidino group, piperazino group, piperidino group, morpholino group, homopiperidino group, heptamethylene-imino group, 4-methylpiperazino group, 4-benzylpiperazino group, 4-hydroxypiperazino group, 4-piperidinopiperidino group and 4-morpholinopiperidino group. Of these, piperazino group, piperidino group, morpholino group, 4-methylpiperazino group, 4-piperidinopiperidino group and 4-morpholinopiperidino group are preferable.

As the lower acylamino group in the present invention, amino groups substituted by a (C1~C5)acyl group are exemplified. Specific examples thereof are acetylamino group, propionylamino group, n-butyrylamino group, isobutyrylamino group, valerylamino group, isovalerylamino group, pivaloylamino group, etc. Of these, acetylamino group is preferable.

As the halogen atom in the present invention, fluorine atom, chlorine atom, bromine atom and iodine atom are exemplified. The halogen atom is preferably a fluorine atom or a bromine atom.

When Z is A(B)CH- in the general formula (1), B is preferably a (C1~C5)alkyl group substituted by one or more groups selected from hydroxyl group, carboxyl group, (C1~C5)alkoxycarbonyl groups, amino group, (C1~C5)alkylamino group, di(C1~C5)alkylamino group, carbamoyl group, saturated heterocyclic group, N-substituted carbamoyl group and G-(C1~C5)alkoxyl group (G is a carboxyl group, an N-substituted carbamoyl group or a hydrogen atom). Of such substituted (C1~C5)alkyl group, (C1~C5)alkyl group substituted by a carboxyl group, a (C1~C5)alkoxycarbonyl group, a di (C1~C5)alkylamino group or a saturated heterocyclic group are especially preferable.

The (C1~C5)alkoxycarbonyl group, (C1~C5)alkyl-amino group, di(C1~C5)alkylamino group and saturated heterocyclic group are the same as those exemplified as the above-mentioned alkoxycarbonyl group, alkylamino group and saturated heterocyclic group. Preferable examples thereof are also the same as in the case of these above-mentioned groups.

As the substituents of the N-substituted carbamoyl group, there are exemplified (C1~C5)alkyl group substituted by a heterocyclic group, such as morpholinoethyl group, 3-picolyl group, 4-picolyl group, etc.

The (C1~C5)alkoxy group of the G-(C1~C5)alkoxy group (G is a carboxyl group, an N-substituted carbamoyl group or a hydrogen atom) are the same as those exemplified as the above-mentioned alkoxy group. Preferable examples thereof are also the same as in the case of the above-mentioned alkoxy group. As the substituent of the N-substituted carbamoyl group for G, there are exemplified (C1~C5)alkyl group substituted by a heterocyclic group, such as morpholinoethyl group, 3-picolyl group, 4-picolyl group, etc., which is the same as the substituent of the N-substituted carbamoyl group as the substituent(s) of the above-mentioned substituted (C1~C5)alkyl group for B.

Specific preferable example of group for B is carboxymethyl group or dimethylamino-2-ethyl group.
Specific preferable example of group for A is hydroxyl group, methyl group or hydrogen atom. Hydrogen atom is especially preferable.

As X of the compound represented by the general formula (1) of the present invention, methyl group, methoxycarbonyl group, acetylamino group, methoxy group, trifluoromethyl group, nitro group, cyano group and halogen atoms are preferable. Trifluoromethyl group is especially preferable. As X', trifluoromethyl group and hydrogen atom are preferable. Hydrogen atom is especially preferable. X and X' are substituents on the benzene ring and their positions are not particularly limited, though the compound is preferably that substituted in the 3-position and 3-trifluoromethyl group is especially preferable.

As Y of the compound represented by the general formula (1) of the present invention, methyl group is especially preferable, and as Y' of the compound, hydrogen atom is especially preferable.

As the compound represented by the general formula (1), there are exemplified the compounds specifically listed in the following tables 1. In the tables, Et indicates an ethyl group, Me a methyl group, Ac an acetyl group, and iPr an isopropyl group.

[Table 1-1]

| Compound No. | Z | Y | Y' | X | X' |
|---|---|---|---|---|---|
| 1 | HO (O=) $CCH_2CH_2$ | Me | H | 3-F | 5-$CF_3$ |
| 2 | HO(O=) $CCH_2CH_2$ | Me | H | 3-$CF_3$ | 5-$CF_3$ |
| 3 | HO (O=) $CCH_2CH_2$ | Me | H | 3-CN | H |
| 4 | HO (O=) $CCH_2CH_2$ | Me | H | 3-Br | H |
| 5 | HO (O=) $CCH_2CH_2$ | Me | H | 3-$NO_2$ | H |
| 6 | HO (O=) $CCH_2CH_2$ | Me | H | 3-Me | H |
| 7 | HO (O=) $CCH_2CH_2$ | Me | H | 3-$CO_2Me$ | H |
| 8 | HO (O=) $CCH_2CH_2$ | Me | H | 3-NHAc | H |
| 9 | HO (O=) $CCH_2CH_2$ | Me | H | 3-F | H |
| 10 | HO (0=) $CCH_2CH_2$ | Me | H | 3-OMe | H |
| 11 | $Et_2NCOCH_2CH_2$ | Me | H | 3-$CF_3$ | H |
| 12 | HO (O=) $CCH_2CH(OH)$ | Me | H | 3-$CF_3$ | H |
| 13 | $EtOCH_2$ | Me | H | 3-$CF_3$ | H |
| 14 | HO (O=) $COCH_2OCH_2$ | Me | H | 3-$CF_3$ | H |
| 15 | Morpholino-$CH_2CH_2NHCOCH_2OCH_2$ | Me | H | 3-$CF_3$ | H |

[Table 1-2]

| Compound No. | Z | Y | Y' | X | X' |
|---|---|---|---|---|---|
| 16 | 3-picolyl-$NHCOCH_2OCH_2$ | Me | H | 3-$CF_3$ | H |
| 17 | 4-picolyl-$NHCOCH_2OCH_2$ | Me | H | 3-$CF_3$ | H |
| 18 | HO (O=) $CCH_2CH_2$ | Me | H | 3-$CF_3$ | H |
| 19 | HO(O=) $CCH_2CH_2CH_2$ | Me | H | 3-$CF_3$ | H |
| 20 | HO (O=) $CCH(OH)CH_2$ | Me | H | 3-$CF_3$ | H |
| 21 | MezNCHzCHzCHz | Me | H | 3-$CF_3$ | H |
| 22 | HO (O=) $CCH_2$ | Me | H | 3-$CF_3$ | H |
| 23 | Morpholino-$CH_2CH_2NHCOCH_2CH_2CH_2$ | Me | H | 3-$CF_3$ | H |
| 24 | 3-picolyl-$NHCOCH_2CH_2CH_2$ | Me | H | 3-$CF_3$ | H |
| 25 | 4-picolyl-$NHCOCH_2CH_2CH_2$ | Me | H | 3-$CF_3$ | H |
| 26 | MeO (O=) $CCH_2CH_2$ | Me | H | 3-CFa | H |
| 27 | EtO (O =) $CCH_2CH_2$ | Me | H | 3-$CF_3$ | H |
| 28 | $EtOCH_2CH_2O$ | Me | H | 3-Me | H |
| 29 | $EtOCH_2CH_2O$ | Me | H | 3-$CO_2Me$ | H |
| 30 | $EtOCH_2CH_2O$ | Me | H | 3-NHAc | H |
| 31 | $EtOCH_2CH_2O$ | Me | H | 3-F | H |
| 32 | $EtOCH_2CH_2O$ | Me | H | 3-OMe | H |
| 33 | $EtOCH_2CH_2O$ | Me | H | 3-Br | H |
| 34 | $MeOCH_2CH_2O$ | Me | H | 3-$NO_2$ | H |

(continued)

| Compound No. | Z | Y | Y' | X | X' |
|---|---|---|---|---|---|
| 35 | $MeOCH_2CH_2O$ | Me | H | 3-CN | H |
| 36 | $MeOCH_2CH_2O$ | Me | H | 3-F | 5-$CF_3$ |
| 37 | $MeOCH_2CH_2O$ | Me | H | 3-$CF_3$ | 5-$CF_3$ |
| 38 | MeOCHzCHzO | Me | H | 3-$CF_3$ | H |
| 39 | Morpholino-$CH_2CH_2O$ | Me | H | 3-$CF_3$ | H |
| 40 | 4-Piperidinopiperidino | Me | H | 3-$CF_3$ | H |
| 41 | 4- (4-Morpholino)piperidino | Me | H | 3-$CF_3$ | H |
| 42 | 4-Methylpiperidino | Me | H | 3-$CF_3$ | H |
| 43 | Morpholino | Me | H | 3-$CF_3$ | H |
| 44 | 4-Methylpiperazino | Me | Me | 3-$CF_3$ | H |
| 45 | 4-Methylpiperazino | iPr | H | 3-$CF_3$ | H |
| 46 | 4-Methylpiperazino | H | H | 3-$CF_3$ | H |
| 47 | Morpholino-$CH_2CH_2NH$ | Me | H | 3-$CF_3$ | H |
| 48 | $HOCH_2CH_2NH$ | Me | H | 3-$CF_3$ | H |
| 49 | $MeOCH_2CH_2CH_2NH$ | Me | H | 3-$CF_3$ | H |
| 50 | EtO (O=) $CCH_2CH_2NH$ | Me | H | 3-$CF_3$ | H |
| 51 | $Me_2NCH_2CH_2NH$ | Me | H | 3-$CF_3$ | H |
| 52 | $Et_2N$ | Me | H | 3-$CF_3$ | H |
| 53 | HO (O=) $CCH_2CH(Me)$ | Me | H | 3-$CF_3$ | H |

Of the compounds listed in the tables, compounds No. 1 to No. 39 are preferable from the viewpoint of antitumor effect, physical properties and the like. Compounds No. 13 to No. 27 are especially preferable. Of these compounds, compounds No. 18 and No. 21 are particularly preferable.

When the 3-phenyltetrahydrocinnolin-5-ol derivative represented by the above general formula (1) of the present invention has stereoisomers, all of these isomers are included in the present invention, though the derivative is preferably a compound in which the relative configurations relating to the carbon atoms at the 5-position and 7-position correspond to syn conformation.

When the 3-phenyltetrahydrocinnolin-5-ol derivative represented by the above general formula (1) has an asymmetric carbon atom, such a compound is present as an optical isomer or a racemic form. The optical isomers, the racemic form, mixtures thereof and the like are included in the present invention, and compounds whose carbon atom at the 5-position is in S-configuration are preferable. In addition, hydrates or solvates of the 3-phenyltetrahydrocinnolin-5-ol derivative represented by the above general formula (1) are also included in the present invention.

As the physiologically acceptable salt of the present invention, there are exemplified salts with mineral acids such as hydrochloric acid, sulfuric acid, etc.; and salts with organic acids such as acetic acid, succinic acid, fumaric acid, maleic acid, citric acid, benzoic acid, tartaric acid, methanesulfonic acid, p-toluenesulfonic acid, etc. When the derivative of the general formula (1) is a compound having a carboxyl group in the molecule, its salt with an alkali metal such as sodium, potassium or the like may be formed. The above-exemplified salts can easily be prepared by a conventional salt-forming reaction.

As the prodrug of the 3-phenyltetrahydrocinnolin-5-ol derivative represented by the general formula (1) or physiologically acceptable salt thereof of the present invention, there are exemplified compounds which are converted to the 3-phenyltetrahydrocinnolin-5-ol derivative represented by the general formula (1) or physiologically acceptable salt thereof of the present invention, for example, by oxidation, reduction, hydrolysis or the like with an enzyme, gastric acid or the like under physiological conditions in a living body (for example, the physiological conditions described in "Development of Medicines Vol. 7, Molecular Design", HIROKAWA-SHOTEN Ltd., 1990, pp. 163-198) to exhibit cytostatic activity and antitumor activity. Specific examples of such prodrugs are compounds (for example, acetylation products and ethyl ester compounds) obtained by the formation of an ester group by the reaction of a hydroxyl or carboxyl group

capable of being present in the substituent Z of the 3-phenyltetrahydrocinnolin-5-ol derivative represented by the general formula (1) or physiologically acceptable salt thereof with a C2~C6 aliphatic carboxylic acid, an aromatic carboxylic acid (e.g. benzoic acid), a C1~C6 aliphatic alcohol or the like; compounds obtained by the formation of an ether group by the reaction of a hydroxyl group capable of being present in the substituent Z with a benzyl halide or the like; and compounds obtained by the formation of an amide group by the reaction of a saturated-heterocyclic, lower-alkylamino or lower-acylamino group capable of being present in the substituent Z with a C2~C6 aliphatic carboxylic acid, an aromatic carboxylic acid (e.g. benzoic acid) or C2~C6 aliphatic carboxylic anhydride (e.g. trifluoroacetic anhydride).

The 3-phenyltetrahydrocinnolin-5-ol derivative, physiologically acceptable salt thereof or prodrug of either of the present invention has cytostatic activity, more specifically cytostatic activity against tumor cells such as mastocarcinoma cells, and hence is effective as a cytostatic agent or an antitumor agent. As the cytostatic or antitumor agent according to the present invention, the 3-phenyltetrahydro-cinnolin-5-ol derivative, physiologically acceptable salt thereof or prodrug of either of the present invention is orally or parenterally administered after being formulated alone or in admixture with an excipient or a carrier into a pharmaceutical composition such as a suspension, an emulsion, an injection, an inhalation, tablets, pills, granules, fine subtilaes, a powder, capsules, an oral solution, a suppository, a percutaneous solution, a percutaneous patch, an ointment, a trans-mucosal solution, a trans-mucosal patch or the like. As the additive such as the excipient or carrier, a pharmaceutically acceptable one is chosen, and its kind and proportion depend on administration route and administration method. For example, in the case of the injection, sodium chloride and sugars (e.g. glucose and mannitol) and the like are preferable. In the case of the compositions for oral administration, starch, lactose, crystalline cellulose, magnesium stearate and the like are preferable. If desired, the above-exemplified pharmaceutical compositions may contain assistants, stabilizers, wetting agents, emulsifiers, buffers and other conventional additives.

Although the content of the compound of the present invention in the pharmaceutical composition is varied depending on the kind of the composition, it is usually 0.1 to 100% by weight, preferably 1 to 98% by weight. For example, the injection preferably contains the active ingredient in an amount of usually 0.1 to 30% by weight, more preferably 1 to 10% by weight. In the case of the compositions for oral administration, the compound of the present invention is used together with additives in the form of tablets, capsules, a powder, granules, a solution, a dry syrup or the like. The capsules, tablets, granules or powder usually contains the active ingredient in an amount of 5 to 100% by weight, preferably 25 to 98% by weight.

The dose is determined depending on, for example, the age, sex, body weight and symptom of a patient and purpose of treatment. For treatment, the compound of the present invention is usually administered in a dose of 0.001 to 100 mg/kg/day in the case of parenteral administration, or 0.01 to 500 mg/kg/day, preferably 0.1 to 100 mg/kg/day, in the case of oral administration, in one portion or 2 to 4 portions.

The compound of the present invention also includes so-called prodrugs of the 3-phenyltetrahydrocinnolin-5-ol derivative represented by the general formula (1) of the present invention which are converted to said derivative, for example, by oxidation, reduction, hydrolysis or the like with an enzyme, gastric acid or the like under physiological conditions in a living body (for example, the physiological conditions described in "Development of Medicines Vol. 7, Molecular Design", HIROKAWA-SHOTEN Ltd., 1990, pp. 163-198) to exhibit antitumor activity.

Processes for producing the 3-phenyltetrahydro-cinnolin-5-ol derivative represented by the above general formula (1) of the present invention are explained below but a process for producing said derivative is not limited thereto.

As an a-halogeno substituted acetophenone derivative represented by the general formula (2) shown below, there are also compounds purchasable from Tokyo Kasei Kogyo Co., Ltd. or the like. It is also possible to obtain the derivative of the general formula (2) by easy halogenation of an acetophenone derivative, which is commercially available or is easily available by a production process based on the content of a well-known reference, by reacting this acetophenone derivative with a halogenating agent such as N-halogenosuccinimde, a single halogen (e.g. bromine or iodine) or a salt (e.g. pyridinium bromide perbromide) in a solvent for reaction such as toluene or tetrahydrofuran in the temperature range of room temperature to heating under reflux.

[Formula 2]

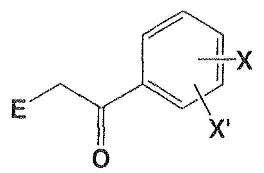

(2)

wherein E is a halogen atom and X and X' are as defined above.

Although there are purchasable compounds as a 1,3-cyclohexanedione derivative represented by the general formula (5) shown below, this derivative is, if necessary, prepared also by reacting a methyl vinyl ketone derivative (3) with a malonic ester derivative (4) in the presence of a metal alkoxide (e.g. sodium methoxide or sodium ethoxide) or a metal hydroxide (e.g. sodium hydroxide or potassium hydroxide) in a solvent such as water, methanol or ethanol at room temperature to temperature at heating under reflux according to the following scheme.

[Formula 3]

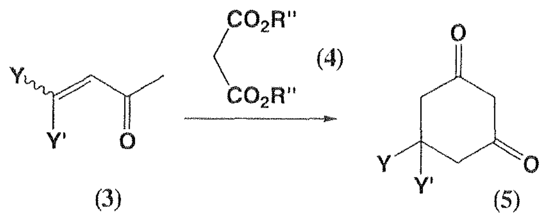

(3) (4) (5)

wherein R" is a lower alkyl group and Y and Y' are as defined above.

Alternatively, when Y' is a hydrogen atom, the derivative of the general formula (5) may be prepared by hydrogenating a resorcinol derivative in an organic solvent such as methanol or tetrahydrofuran in the presence of a catalyst such as platinum or palladium.

The above-mentioned 1,3-cycloalkanedione derivative is converted to a compound represented by the general formula (7) by reacting said derivative with the compound of the above general formula (2) in an organic solvent (e.g. dimethyl sulfoxide, dichloromethane, chloroform, tetrahydrofuran, methanol or ethanol) in the presence of a base (e.g. sodium hydride, sodium hydroxide, potassium hydroxide, potassium carbonate, cesium carbonate, sodium methoxide or sodium ethoxide) at room temperature to temperature at heating under reflux.

[Formula 4]

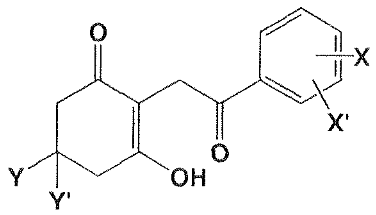

(7)

wherein X, X', Y and Y' are as defined above.

A 4,6,7,8-hexahydro-1H-cinnolin-5-one derivative represented by the general formula (8) may be obtained by reacting the compound of the general formula (7) with hydrazine hydrochloride in an organic solvent (e.g. methanol or ethanol) in the presence of a base (e.g. triethylamine or pyridine) at room temperature to temperature at heating under reflux.

[Formula 5]

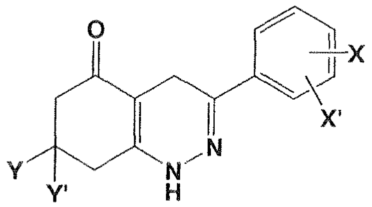

(8)

wherein X, X', Y and Y' are as defined above.

In addition, a compound represented by the following general formula (9) may be obtained by oxidizing the compound of the general formula (8) by heating under reflux in the presence or absence of a metal catalyst (e.g. palladium or platinum) in an organic solvent (e.g. pyridine, triethylamine, methanol, ethanol, acetone, acetic acid or tetrahydrofuran), or reacting the compound of the general formula (8) with an oxidizing agent such as cerium (IV) ammonium nitrate or 2,3-dichloro-5,6-dicyano-p-benzoquinone in acetone, methanol, tetrahydrofuran or a mixed solvent thereof.

[Formula 6]

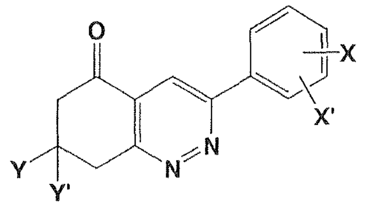

(9)

wherein X, X', Y and Y' are as defined above.

Furthermore, the compound of the general formula (9) may be converted to 3-phenyltetrahydrocinnolin-5-ol, a compound represented by the general formula (10) by reacting the compound of the general formula (9) with a reducing agent (e.g. sodium tetrahydroborate, lithium aluminum hydride or lithium tri-tert-butoxyaluminum hydride) in an organic solvent (e.g. tetrahydrofuran, methanol or ethanol) in the temperature range of ice-cooling to room temperature.

[Formula 7]

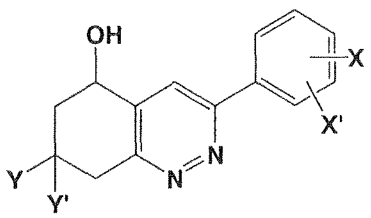

(10)

wherein X, X', Y and Y' are as defined above.

The compound of the general formula (10) thus obtained may be converted to a compound of the above general formula (1) in which Z is L(L')N-, by reacting the compound of the general formula (10) with phenyl chloroformate or a substituted phenyl chloroformate such as 4-nitrophenyl chloroformate in an organic solvent (e.g. tetrahydrofuran, dichloromethane or toluene) in the presence of a base (e.g. triethylamine or pyridine), and then reacting the reaction product

with any of various amine compounds (L(L')NH).

In addition, the compound of the general formula (10) may be converted to a compound of the above general formula (1) in which Z is O-M, by reacting the compound of the general formula (10) with a substituted alkyl chloroformate (Cl-COO-M) in the presence of the same solvent and base as above.

Furthermore, the compound of the general formula (10) may be converted to a compound of the above general formula (1) in which Z is A(B) CH-, by reacting the compound of the general formula (10) with an acid anhydride (e.g. succinic anhydride or glutaric anhydride) or a substituted lower acyl halide (e.g. methoxyacetyl chloride) in an organic solvent (e.g. tetrahydrofuran, acetonitrile, dichloromethane or pyridine), or reacting the compound of the general formula (10) with a substituted lower fatty acid such as 4-dimethylaminobutyric acid or mono-t-butyl malonate in the presence of dimethylaminopyridine or the like by the use of a dehydrating-condensation agent such as dicyclohexylcarbodiimide or N-ethyl-N'-3-dimethylaminopropylcarbodiimide hydrochloride (EDC).

In any of the above production processes, it is also possible to carry out the reaction by, if necessary, using a protective group used in a usual organic reaction (described, for example, in Green, T.W. et al. "PROTECTIVE GROUPS IN ORGANIC SYNTHESIS", 2nd. Ed., WILEY·INTERSCIENCE (USA)), and carry out deprotection when the protective group becomes unnecessary, to obtain a desired compound.

For the isolation and purification of a desired compound from the reaction mixture obtained in each of the production processes described above, conventional solvent extraction, concentration, distillation, recrystallization, chromatography and the like may be properly employed if necessary.

A physiologically acceptable salt or prodrug of the compound of the general formula (1) obtained by each of the above-mentioned production processes can easily be obtained from the compound of the general formula (1) by a per se well-known method.

The present invention is concretely illustrated below with reference to production examples and test examples as working examples, which should not be construed as limiting the scope of the invention.

In the working examples, ESI is an abbreviation for Electron Spray Ionization, which is an ionization method in molecular weight measurement.

Reference Example 1.

Synthesis of 2-bromo-3'-trifluoromethylacetophenone

Pyridinium bromide perbromide (135.4 g, 0.423 mol) was added to a solution of commercial 3'-trifluoromethyl-acetophenone (79.6 g, 0.423 mol) in toluene (423 ml) under ice-cooling, and the resulting mixture was stirred for 5 hours while being heated to room temperature. The reaction mixture was ice-cooled again and 400 ml of distilled water was added dropwise thereto to terminate the reaction, and then the resulting mixture was separated. The toluene layer was washed with 400 ml of a saturated aqueous sodium hydrogencarbonate solution and dried over anhydrous sodium sulfate, and the toluene was concentrated under reduced pressure, followed by distillation under reduced pressure, whereby the desired compound (92.35 g, 81.7%) was obtained.

$^1$H-NMR(200MHzFT, TMS, $CDCl_3$)

4.46(2H,s),7.66(1H,brt,J=7.9Hz),7.88(1H,brd,J=7.6Hz),8. 19(1H,brd,J=7.5Hz),8.25(1H,brs)

b.p. 92°C/3mmHg

Reference Example 2

Synthesis of 3-hydroxy-5-methyl-2-[2-oxo-2-(3-trifluoromethylphenyl)ethyl]cyclohex-2-enone

Potassium carbonate (32.9 g, 0.238 mol) was added to a solution of the 2-bromo-3'-trifluoromethylacetophenone obtained in Reference Example 1 (63.5 g, 0.238 mol) and 5-methyl-1,3-cyclohexanedione (30 g, 0.238 mol) in chloroform (240 ml), and the resulting mixture was stirred overnight at room temperature. The reaction mixture was filtered and the white solid obtained was suspended in distilled water (300 ml), followed by adding dropwise thereto concentrated hydrochloric acid (30 ml) under ice-cooling. The resulting mixture was extracted with ethyl acetate (700 ml) and ethanol (50 ml) and the extract solution was dried over anhydrous sodium sulfate. The dried extract solution was filtered and the organic layer thus obtained was concentrated under reduced pressure. To the resulting residue was added ethyl acetate (200 ml), followed by suspending and stirring at room temperature for 4 hours. The crystals were collected by filtration to obtain the desired compound (25.7 g, 34.6%).

$^1$H-NMR(200MHzFT,TMS,$CDCl_3$)

1.06(3H,d,J=5.9Hz),1.98-2.63(5H,complex),3.77(1H,d,J-13.6Hz),4.29(1H,d, J=13.6Hz),7.63(1H,brt,J=7.6Hz),7.87(1H, brd,J=7.8Hz),

8.43-8.52(2H,complex),9.64(1H,s)

MS(ESI,POS) m/z 313 $[M+H]^+$

Reference example 3

Synthesis of 7-methyl-3-(3-trifluoromethylphenyl)-4,6,7,8-tetrahydro-1H-cinnolin-5-one

Hydrazine hydrochloride (177 mg, 1.7 mmol.) and triethylamine (0.49 ml, 3.5 mmol) were added to a solution of the 3-hydroxy-5-methyl-2-[2-oxo-2-(3-trifluoromethyl-phenyl)ethyl]cyclohex-2-enone obtained in Reference Example 2 (438.7 mg, 1.4 mmol) in ethanol (14 ml), and the resulting mixture was stirred at room temperature for 3 hours. The reaction mixture was concentrated and the resulting residue was purified by a silica gel column chromatography (methylene chloride/methanol = 30/1) to obtain the desired compound (100.9 mg, 23.3%).
$^1$H-NMR (200MHzFT, TMS, $CDCl_3$) 1.13(3H, d, J=5.9Hz), 2.00-2.60 (5H,complex), 3.27 (1H, d, J=9.3Hz), 3.57 (1H, d, J=9.3Hz), 7.49(1H, brs), 7.54 (1H, brd, J=7.9Hz), 7.65 (1H, brd, J=7.7Hz),
7.94 (1H, brd, J=7.8Hz), 8.08 (1H, brs)
MS(ESI, POS) m/z 309 $[M+H]^+$

Reference Example 4

Synthesis of 7-methyl-3-(3-trifluoromethylphenyl)-7,8-dihydro-6H-cinnolin-5-one

p-Toluenesulfonic acid monohydrate (84 mg, 0.44 mmol) was added to a solution of the 7-methyl-3-(3-trifluoromethylphenyl)-4,6,7,8-tetrahydro-1H-cinnalin-5-one obtained in Reference Example 3 (136.2 mg, 0.44 mmol) in pyridine (1 ml), and the resulting mixture was stirred at room temperature for 3 days. The reaction mixture was concentrated and the resulting residue was purified by a silica gel column chromatography (methylene chloride/methanol = 30/1) to obtain the desired compound (89.0 mg, 66.1%).
$^1$H-NMR(200MHzFT,TMS,$CDCl_3$)
1.28(3H,d,J=1.3Hz),2.40-2.62(2H,complex),2.80-2.89(1H,m),2.90-3.19(1H,m),3.55-3.70(1H,m), 7.68(1H,brt,J=7.7Hz), 7.74(1H,brd,J-7.7Hz),8.29(1H,s),
8.34(1H,brd,J=7.3Hz),8.44(1H,brs)
MS(ESI,POS) m/z 307 $[M+H]^+$

Reference Example 5

Synthesis of 7-methyl-3-(3-trifluoromethylphenyl)-5,6,7,8-tetrahydrocinnolin-5-ol

Sodium tetrahydroborate (123.5 mg, 3.28 mmol) was added to a solution of the 7-methyl-3-(3-trifluoromethylphenyl)-7,8 dihydro-6H-cinnolin-5-one obtained in Reference Example 4 (1 g, 3.28 mmol) in ethanol (20 ml) under ice-cooling, and the resulting mixture was stirred at room temperature for 1 hour. After completion of the reaction, the reaction was quenched with a 1-normal aqueous potassium hydrogensulfate solution (15 ml), followed by extraction with ethyl acetate (100 ml). The extract solution was dried over anhydrous sodium sulfate and then the drying agent was filtered off. The organic layer was concentrated under reduced pressure and the resulting residue was purified by a silica gel column chromatography (hexane/ethyl acetate = 1/1 to 1/2) to obtain the desired compound (918 mg, 90.9%) as a light-yellow solid. The syn/anti ratio of this compound was about 9/1 as measured by HPLC (high performance liquid chromatography).
$^1$H-NMR (200MHzFT, TMS, $CDCl_3$)
1.22(3H,d,J=6.6Hz),1.51(1H,q,J=12.2Hz),1.88-2.44(1H,m), 2.24-2.42(1H,m),2.73(1H,ddd,J=1.1,11.7,18.0Hz), 3.41 (1H,ddd,J=1.8,5.2,17.8Hz),4.90(1H,q,J=5.8,11.3Hz), 7.62(1H,t,J=7.7Hz),7.73(1H,d,J=7.8Hz),8.14(1H,d, J=1.1Hz), 8.29(1H,d,J=8.0Hz),8.34(1H,s)
MS(ESI,POS) m/z 309 $[M+H]^+$

Reference Example 6

Synthesis of (2,2-dimethyl-5-oxo-[1,3]dioxolan-4-yl)-acetic acid

Pyridinium p-toluenesulfonate (216 mg, 0.86 mmol) was added to a solution of malic acid (1.34 g, 10 mmol) in 2,2-dimethoxypropane (4.5 ml), and the resulting mixture was stirred at room temperature for 32 hours. The reaction mixture was concentrated and then purified by a silica gel column chromatography (ethyl acetate) to obtain the desired compound (1.58 g, 90.7%) as a white solid.

$^1$H-NMR(200MHzFT,TMS,$CDCl_3$) 1.56(3H,s),1.63(3H,s),2.85(1H,dd,J=6.3,17.2Hz), 3.01(1H,dd,J=4.0,17.2Hz),4.07 (1H,dd,J=4.0,6.3Hz), 7.20-8.1(1H,br)

Reference Example 7

Synthesis of 7-methyl-5-(phenyloxycarbonyl)oxy-3-(3-trifluoromethylphenyl)-5,6,7,8-tetrahydrocinnoline

Pyridine (1.6 ml) was added to a suspension of the 7-methyl-3-(3-trifluoromethylphenyl)-5,6,7,8-tetrahydrocinnolin-5-ol obtained in Reference Example 5 (3.11 g) in toluene (20 ml) and phenyl chloroformate (1.5 ml) was added dropwise thereto. After overnight stirring at room temperature, the reaction mixture was transferred into a separatory funnel with tap water and toluene and washed 5 times with tap water. After drying over anhydrous sodium sulfate, the organic layer was evaporated to obtain the desired compound (2.89 g, 67%).
MS(ESI, POS) m/z 429 $[M+H]^+$

Reference Example 8

Synthesis of 5-(2-chloroethoxycarbonyl)oxy-7-methyl-3-(3-trifluoromethylphenyl)-5,6,7,8-tetrahydrocinnoline

N,N,N',N'-tetramethylethylenediamine (0.096 ml, 0.6 mmol) and 2-chloroethyl chloroformate (0.12 ml, 1.2 mmol) were added to a solution of the 7-methyl-3-(3-trifluoro-methylphenyl)-5,6,7,8-tetrahydrocinnolin-5-ol obtained in Reference Example 5 (308.3 mg, 1 mmol) in dichloromethane (2 ml) under ice-cooling, and the reaction was carried out at 0°C for 1 hour and then carried out overnight at room temperature. A saturated aqueous sodium hydrogencarbonate solution was added to the reaction mixture to effect separation. The resulting organic layer was washed with a saturated aqueous sodium chloride solution and then dried over anhydrous sodium sulfate. The drying agent was filtered off and the residue was concentrated under reduced pressure. To the resulting residue were added 2-propanol and hexane to effect crystallization. The crystals were collected by filtration to obtain the desired compound (343 mg, 82.7%).
$^1$H-NMR(200MHzFT,TMS,$CDCl_3$) 1.26(3H,d,J=6.6Hz),1.64(1H,dd,J=12.2,23.4Hz), 2.08-2.30(1H,m),2.40-2.57(1H,m), 2.83(1H,dd,J=11.5,17.6Hz),3.53(1H,dd,J=3.7,18.3Hz), 3.80(1H,t,J=5.3Hz),4.54(1H,dt,J=2.2,5.5Hz), 5.89(1H,dd, J=6.0,10.8Hz),7.66(1H,t,J=7.8Hz), 7.77(1H,d,J=7.5Hz),7.88(1H,s),8.28(1H,d,J=7.9Hz), 8.34(1H,s)
MS(ESI,POS) m/z 415,417$[M+H]^+$

Example 1

Synthesis of 5-ethoxyacetyloxy-7-rnethyl-3-(3-trifluoromethylphenyl)-5,6,7,8-tetrahydrocinnoline (compound No. 13)

The 7-methyl-3-(3-trifluoromethylphenyl)-5,6,7,8-tetrahydrocinnolin-5-ol obtained in Reference Example 5 (308.3 mg, 1 mmol) was dissolved in a mixed solvent of N,N-dimethylformamide/1-methyl-2-pyrrolidone (2 ml/0.4 ml), followed by adding thereto EDC (288 mg, 1.5 mmol), N,N-dimethylaminopyridine (12.2 mg, 0.1 mmol) and ethoxyacetic acid (0.14 ml, 0.045 mmol), and the resulting mixture was stirred overnight at room temperature. After completion of the reaction, distilled water was added to the reaction mixture, followed by extraction with ethyl acetate. The resulting organic layer was washed successively with a 10% sodium hydrogensulfate solution, a saturated aqueous sodium hydrogencarbonate solution and a saturated aqueous sodium chloride solution. After the washed organic layer was dried over anhydrous sodium sulfate, the drying agent was filtered off and the organic layer thus obtained was concentrated under reduced pressure to obtain the desired compound (390 mg, 98.8%) as white crystals.
$^1$H-NMR(200MHzFT,TMS,$CDCl_3$)
1.24(3H,d,J=6.6Hz),1.30(3H,t,J=7.0Hz),1.56(1H,dd, J=12.3,23.2Hz),2.05-2.33(1H,m),2.34-2.52(1H,m), 2.81(1H,dd, J=11.7,18.0Hz),3.46(1H,dd,J=4.8,18.0Hz), 3.68(2H,q,J=7.0Hz),4.25(2H,d,J=4.2Hz),6.13(1H,dd, J=6.1,10.8Hz),7.64 (1H,t,J=7.8Hz),7.69(1H,s), 7.76(1H,d,J=7.8Hz),8.24(1H,d,J=7.6Hz),8.32(1H,s)
MS (FAF3, POS) m/z 395$[M+H]^+$

Example 2

Synthesis of 5-(carboxymethoxyacetyl)oxy-7-methyl-3-(3-trifluoromethylphenyl)-5,6,7,8-tetrahydro-cinnoline (compound No. 14)

Diglycolic anhydride (580 mg, 5 mmol) was added to a solution of the 7-methyl-3-(3-trifluoromethylphenyl)-5,6,7,8-tetrahydrocinnolin-5-ol obtained in Reference Example 5 (308.3 mg, 1 mmol) in pyridine (2.5 ml), and the resulting mixture was stirred at room temperature for 1 hour. After completion of the reaction, 3 ml of toluene was added to the

reaction mixture and the resulting mixture was concentrated under reduced pressure. To the resulting residue were added 15 ml of ethyl acetate and 15 ml of distilled water, and the resulting mixture was stirred for 30 minutes and then separated. The resulting organic layer was washed with a saturated aqueous sodium chloride solution and dried over anhydrous sodium sulfate. The drying agent was filtered off and the residue was concentrated under reduced pressure. To the resulting residue were added ethyl acetate and 2-propanol, followed by suspending and stirring at room temperature for 30 minutes. The crystals were collected by filtration to obtain the desired compound (369 mg, 87%) as white crystals.
$^1$H-NMR (200MHzFT, TMS, $CDCl_3$)
1.23 (3H, t, J=7.0Hz), 1.55 (1H, dd, J=12.3, 23.2Hz), 2.07-2.30 (1H,m), 2.35-2.50 (1H,m), 2.79 (1.H, dd, J-11.4, 17.2Hz), 3.45(1H, ddd, J=1.7, 5.0, 17.6Hz), 4.35 (2H,s), 4.42 (2H,d, J=5.4Hz), 6.13 (1H, dd, J=6.2, 10.9Hz.), 7.64 (1H, t, J=7.8Hz), 7.71-7.80 (1H,m), 7.73 (1H,s), 8.23 (1H, d, J=7.7Hz), 8.32 (1H,s)
MS (ESI, POS) m/z 425 $[M+H]^+$

Example 3

Synthesis of 5-(2-methoxyethoxycarbonyl)oxy-7-methyl-3-(3-trifluoromethylphenyl)-5, 6,7,8-tetrahydro-cinnoline (compound No. 38)

N,N,N',N'-tetramethylethylenediamine (0.096 ml, 0.6 mmol) and 2-methoxyethyl chloroformate (0.14 ml, 1.2 mmol) were added to a solution of the 7-methyl-3-(3-trifluaromethylphenyl)-5,6,7,8-tetrahydrocinnolin-5-ol obtained in Reference Example 5 (308.3 mg, 1 mmol) in dichloromethane (2 ml) under ice-cooling, and the reaction was carried out at 0°C for 1 hour and then carried out overnight at room temperature. A saturated aqueous sodium hydrogen-carbonate solution was added to the reaction mixture to effect separation. The resulting organic layer was washed with a saturated aqueous sodium chloride solution and then dried over anhydrous sodium sulfate. The drying agent was filtered off and the residue was concentrated under reduced pressure. To the resulting residue were added 2-propanol and hexane to effect crystallization. The crystals were collected by filtration to obtain the desired compound (300 mg, 73.2%).
$^1$H-NMR(200MHzFT,TMS,$CDCl_3$)
1.24(3H,d,J=6.5Hz),1.60(1H,q,J=12.3Hz),2.02-2.28(1H,m), 2.38-2.55(1H,m),2.80(1H,dd,J=11.6,17.9Hz),3.31-3.53 (1H,m),3.42(3H,s),3.69(2H,t,J=4.6Hz),4.32-4.52(2H,m),5.86(1H,dd,J=6.0,10.8Hz),7.65(1H,t,J=7.7Hz), 7.76(1H,d, J=7.7Hz),8.28(1H,brd,J=7.7Hz),8.35(1H,brs) MS(ESI,POS) m/z 411 $[M+H]^+$

Example 4

Synthesis of 5-(3-carboxypropanoyl)oxy-7-methyl-3-(3-trifluoromethylphenyl)-5,6,7,8-tetrahydrocinnoline (compound No.18)

Succinic anhydride (1 g, 10 mmol) was added to a solution of the 7-methyl-3-(3-trifluoromethylphenyl)-5,6,7,8-tetrahydrocinnolin-5-ol obtained in Reference Example 5 (616 mg, 2 mmol) in pyridine (10 ml), and the resulting mixture was stirred at 37°C for 48 hours. After completion of the reaction, 10 ml of toluene was added to the reaction mixture and the resulting mixture was concentrated under reduced pressure. To the resulting residue were added 30 ml of ethyl acetate and 30 ml of distilled water, and the resulting mixture was stirred for 1 hour and then separated. The resulting organic layer was washed with a saturated aqueous sodium chloride solution and dried over anhydrous sodium sulfate. The drying agent was filtered off and the residue was concentrated under reduced pressure. To the resulting residue were added ethyl acetate (5 ml) and 2-propanol (30 ml), followed by suspending and stirring at room temperature for 30 minutes. The crystals were collected by filtration to obtain the desired compound (381 mg, 49%) as white crystals.
$^1$H-NMR (200MHzFT, TMS, $CDCl_3$)
1.20(3H, d, J=6.6Hz), 1.54 (1H,q, J=11.9Hz), 2.00-2.40 (2H, m), 2.60-2.94 (5H, m), 3.41. (1H, dd, J=4.1, 17.9Hz), 6.07 (1H, dd, J=6.0, 10.9Hz), 7.54 (1H, t, J=7.7Hz), 7.68 (1H, d, J=7.7Hz), 7.83 (1H,s), 8.23 (1H, d, J=7.8Hz), 8.30 (1H, s)
MS (ESI, POS) m/z 409 $[M+H]^+$

Example 5

Synthesis of 5-(4-carboxybutanoyl)oxy-7-methyl-3-(3-trifluoromethylphenyl)-5,6,7,8-tetrahydrocinnoline (compound No. 19)

The desired compound was obtained by carrying out reaction in the same manner as in Example 2 except for using glutaric anhydride in place of diglycolic anhydride.
$^1$H-NMR(200MHzFT, TMS, $CDCl_3$) 1.22(3H,d,J=6.6Hz),1.50(1H,q,J=11.8Hz),1.98-2.26(3H,m),2.29-2.88(6H,m),3.43(1H,dd,J=5.1,18.0Hz), 6.03(1H,dd,J=6.1,10.7Hz),7.63(1H,t,J=7.7Hz), 7.71(1H,s),7.72(1H,d,J=7.7Hz),8.22.(1H,d, J=7.6Hz), 8.33(1H,s)
MS(ESI,POS) m/z 423 $[M+H]^+$

Example 6

Synthesis of 5-[(3-carboxy-3-hydroxy)propanoyl]oxy-7-methyl-3-(3-trifluoromethylphenyl)-5,6,7,8-tetrahydrocinnoline (compound No. 20)

A colorless syrup (280 mg, 60.3 %) obtained by carrying out treatment in the same manner as in Example 1 except for using the (2,2-dimethyl-5-oxo-[1,3]dioxolan-4-yl)-acetic acid obtained in Reference Example 6 in place of ethoxyacetic acid, was dissolved in a mixture of tetrahydrofuran, distilled water and acetic acid (14 ml each), and the resulting solution was stirred at room temperature for 40 hours. The reaction solution was concentrated under reduced pressure and the resulting residue was purified by a silica gel column chromatography (hexane/ethyl acetate = 1/1 to 0/1) and then crystallized from ethyl acetate/hexane to obtain the desired compound (188 mg, 73.5%) as white crystals.
MS (ESI, POS) m/z 425 $[M+H]^+$

Example 7

Synthesis of 7-methyl-5-(4-piperidinopiperidin-1-yl-carbonyl)oxy-3-(3-trifluoromethylphenyl)-5,6,7,8-tetrahydrocinnoline (compound No. 40)

4-Piperidinopiperidine (170 mg) was added to a solution of the 7-methyl-5-(phenyloxycarbonyl)oxy-3-(3-trifluoromethylphenyl)-5,6,7,8-tetrahydrocinnoline obtained in Reference Example 7 (316.0 mg) in tetrahydrofuran (1.5 ml), and stirred overnight. The reaction mixture was transferred into a separatory funnel with ethyl acetate and washed successively with tap water, a saturated aqueous sodium hydrogencarbonate solution and a saturated aqueous sodium chloride solution. After drying over anhydrous sodium sulfate, the organic layer was evaporated and to the resulting residue was added ethyl acetate/hexane (1/1). The solid precipitated was collected by filtration to obtain the desired compound (420.9 mg, quantitative).
MS (ESI, POS) m/z 429 $[M+H]^+$

Example 8

Synthesis of 7-methyl-5-[4-(4-morpholino)piperidin-1-yl-carbonyl]oxy-3-(3-trifluoromethylphenyl)-5,6,7,8-tetrahydrocinnoline (compound No. 41)

The desired compound (364.0 mg, quantitative) was obtained by carrying out the same procedure as in Example 7 except for using the 7-methyl-5-(phenyloxycarbonyl)oxy-3-(3-trifluoromethylphenyl)-5,6,7,8-tetrahydrocinnoline obtained in Reference Example 7 (307.3 mg) and 4-(4-morpholino)piperidine (227 mg).
MS(ESI, POS) m/z 505 $[M+H]^+$

Example 9

Synthesis of 7-methyl-5-(4-methylpiperazin-1-yl-carbonyl)oxy-3-(3-trifluoromethylphenyl)-5,6,7,8-tetrahydrocinnoline (compound No. 42)

The desired compound (252.4 mg, 81%) was obtained by carrying out the same procedure as in Example 7 except for using the 7-methyl-5-(phenyloxycarbonyl)oxy-3-(3-trifluoromethylphenyl)-5,6,7,8-tetrahydrocinnoline obtained in Reference Example 7 (305.4 mg) and 4-methylpiperazine (0.1 ml). MS(ESI, POS) m/z 435 $[M+H]^+$

Example 10

Synthesis of 7-methyl-5-(morphholin-4-yl-carbonyl)oxy-3-(3-trifluoromethylphenyl) -5,6,7,8-tetrahydrocinnoline hydrochloride (compound No. 43)

The same procedure as in Example 7 was carried out except for using the 7-methyl-5-(phenyloxycarbonyl)oxy-3-(3-trifluoromethylphenyl)-5,6,7,8-tetrahydrocinnoline obtained in Reference Example 7 (282.6 mg) and morpholine (0.1 ml). Thereafter, to the thus obtained product was added 4-normal hydrogen chloride/1,4-dioxane (0.2 ml), followed

by evaporation. To the resulting residue was added ethyl acetate (6 ml) and the resulting mixture was stirred at 40°C for 1 hour and then stirred overnight at room temperature. The solid precipitated was collected by filtration to obtain the desired compound (1.95.4 mg, 64%).
MS(ESI, POS) m/z 422 [M+H-HCl]$^+$

Example 11

Synthesis of 5-(4-dimethylaminobutanoyl)oxy-7-methyl-3-(3-trifluoromethylphenyl)-5,6,7,8-tetrahydro-cinnoline (compound No. 21)

The 7-methyl-3-(3-trifluoromethylphenyl)-5,6,7,8-tetrahydrocinnolin-5-ol obtained in Reference Example 5 (318 mg) and 4-dimethylaminobutyric acid hydrochloride (529.2 mg) were dissolved in N,N-dimethylformamide (4.1 ml), followed by adding thereto dicyclohexylcarbodiimide (638 mg), and the resulting mixture was stirred overnight at room temperature. Ethyl acetate was added thereto and the solid precipitated was filtered off. Then, the filtrate was transferred into a separatory funnel and washed successively with tap water, a saturated aqueous sodium hydrogencarbonate solution and a saturated aqueous sodium chloride solution. After drying over anhydrous sodium sulfate, the organic layer was evaporated. Ethyl acetate was added to the residue and the solid precipitated was collected by filtration to obtain the desired compound (430.4 mg, 99%).
MS(ESI, POS) m/z 422 [M+H]$^+$

Example 12

Synthesis of 5-(2-carboxyacetyl)oxy-7-methyl-3-(3-trifluaromethylphenyl)-5, 6, 7, 8-tetrahydrocinnoline hydrochloride (compound No. 22)

The 7-methyl-3-(3-trifluoromethylphenyl)-5,6,7,8-tetrahydrocinnolin-5-ol obtained in Reference Example 5 (552 mg) and mono-t-butyl malonate (286.7 mg) were dissolved in N,N-dimethylformamide (5 ml), followed by adding thereto EDC (1.03 g), and the resulting mixture was stirred overnight at room temperature. The reaction mixture was transferred into a separatory funnel with ethyl acetate and tap water and washed successively with tap water, a saturated aqueous sodium hydrogencarbonate solution and a saturated aqueous sodium chloride solution. After drying over anhydrous sodium sulfate, the organic layer was evaporated. To the residue was added 4-normal hydrogen chloride/1,4-dioxane (3 ml), and stirred overnight and then the reaction mixture was evaporated. To the resulting residue was added ethyl acetate and the solid precipitated was collected by filtration to obtain the desired compound (158.0 mg, 22%).
MS(ESI, POS) m/z 395 [M+H-HCl]$^+$

Example 13

Synthesis of 5-[3-[2-(morpholin-4-yl)ethyl]amino-carbonylpropanoyl]oxy-7-methyl-3-(3-trifluoromethylphenyl)-5,6,7,8-tetrahydrocinnoline (compound No. 23)

2-(4-Morpholino)ethylamine (0.02 ml, 0.15 mmol), EDC (28.8 mg, 0.15 mmol) and 1-hydroxybenzotriazole (16.2 mg, 0.12 mmol) were added to a solution of the 5-(3-carboxypropanoyl)oxy-7-methyl-3-(3-trifluoromethylphenyl)-5,6,7,8-tetrahydrocinnoline obtained in Example 4 (40.8 mg, 0.1 mmol) in N,N-dimethylformamide (1 ml), and the reaction was carried out overnight at room temperature. Ethyl acetate and distilled water were added to the reaction mixture to effect extraction and then the organic layer was washed successively with a 1-normal aqueous sodium hydrogensulfate solution, a saturated aqueous sodium hydrogencarbonate solution and a saturated aqueous sodium chloride solution. The washed organic layer was concentrated under reduced pressure to obtain the desired compound (12.4 mg, 24%) as a white solid. MS(ESI, POS) m/z 521 [M+H]$^+$

Example 14

Synthesis of 5-[3-(pyridin-3-ylmethyl)amino-carbonylpropanoyl]oxy-7-methyl-3-(3-trifluoromethylphenyl)-5,6,7,8-tetrahydrocinnoline (compound No. 24)

The desired compound was obtained by carrying out treatment in the same manner as in Example 13 except for using 3-picolylamine in place of 2-(4-morpholino)ethylamine.
MS (ESI, POS) m/z 499 [M+H]$^+$

Example 15

Synthesis of 5-[3-(pyridin-4-ylmethyl)amino-carbonylpropanoyl]oxy-7-methyl-3-(3-trifluoromothylphenyl) -5,6,7,8-tetrahydrocinnoline (compound No.25)

The desired compound was obtained by carrying out treatment in the same manner as in Example 13 except for using 4-picolylamine in place of 2-(4-morpholino)ethylamine.
MS(ESI, POS) m/z 499 $[M+H]^+$

Example 16

Synthesis of 5-[(2-(morphol-in-4-yl)ethyl)aminocarbonylmethoxyacetyl]oxy-7-methyl-3-(3-trifluoromethylphenyl)-5,6,7,8-tetrahydrocinnoline (compound No. 15)

The desired compound was obtained by carrying out treatment in the same manner as in Example 13 except for using the 5-carboxymethoxyacetyloxy-7-methyl-3-(3-trifluoromethylphenyl)-5,6,7,8-tetrahydrocinnoline obtained in Example 2 in place of the 5-(3-carboxypropanoyl)oxy-7-methyl-3-(3-trifluoromethylphenyl)-5,6,7,8-tetrahydrocinnoline,
MS (ESI, POS) m/z 610 $[M+H]^+$

Example 17

Synthesis of 5-[(pyridin-3-ylmethyl)amino-carbonylmethoxyacetyl]oxy-7-methyl-3-(3-trifluoromethyl-phenyl)-5,6,7,8-tetrahydrocinnoline (compound No. 16)

The desired compound was obtained by carrying out treatment in the same manner as in Example 13 except for using the 5-carboxymethoxyacetyloxy-7-methyl-3-(3-trifluoromethylphenyl)-5,6,7,8-tetrahydrocinnoline obtained in Example 2 in place of the 5-(3-carboxypropanoyl)oxy-7-methyl-3-(3-trifluoromethylphenyl)-5,6,7,8-tetrahydrocinnoline, and 3-picolylamine in place of 2-(4-morpholino)ethylamine. MS(ESI, POS) m/z 515 $[M+H]^+$

Example 18

Synthesis of 5-[(pyridin-4-ylmethyl)amino-carbonylmethoxyacetyl]oxy-7-methyl-3-(3-trifluoromethyl-phenyl)-5,6,7,8-tetrahydrocinnoline (compound No. 17)

The desired compound was obtained by carrying out treatment in the same manner as in Example 13 except for using the 5-carboxymethoxyacetyloxy-7-methyl-3-(3-trifluoromethylphenyl)-5,6,7,8-tetrahydrocinnoline obtained in Example 2 in place of the 5-(3-carboxypropanoyl)oxy-7-methyl-3-(3-trifluoromethylphenyl)-5,6,7,8-tetrahydrocinnoline, and 4-picolylamine in place of 2-(4-morpholino)ethylamine. MS (ESI, POS) m/z 515 $[M+H]^+$

Example 19

Synthesis of 5-(2-(morpholino-4-yl)ethoxycarbonyl)oxy-7-methyl-3-(3-trifluoromethylphenyl)-5,6,7,8-tetrahydrocinnoline (compound No. 39)

N,N-diisopropylethylamine (0.07 ml) and morpholine (0.05 ml, 0.58 mmol) were added to a solution (1 ml) of the 5-(2-chloroethoxycarbonyl)oxy-7-methyl-3-(3-trifluoromethylphenyl)-5,6,7,8-tetrahydrocinnoline obtained in Reference Example 8 (160 mg, 0.39 mmol) in N,N-dimethyltiormamide, and the resulting mixture was stirred at room temperature for 3 days. The reaction mixture was concentrated under reduced pressure and the resulting residue was purified by a silica gel column chromatography (dichloromethane/methanol = 10/1) to obtain the desired compound (2.5 mg, 1.4%).
MS (ESI, POS) m/z 467 $[M+H]^+$

Example 20

Synthesis of 5-(3-methoxycarbonylpropanoyl)oxy-7-methyl-3-(3-trifluoromethylphenyl)-5,6,7,8-tetrahydro-cinnoline (compound No. 26)

The 5-(3-carboxypropanoyl)oxy-7-methyl-3-(3-trifluoromethylphenyl)-5,6,7,8-tetrahydrocinnoline obtained in Example 4 was dissolved in tetrahydrofuran, followed by adding thereto an excessive amount of a solution of trimethylsilyldiazomethane in hexane, and the reaction was carried out under ice-cooling for 1 hour. The reaction mixture was concentrated under reduced pressure and the resulting residue was purified by a silica gel column chromatography (hexane/ethyl acetate = 1/1) to obtain the desired compound.
$^1$H-NMR(200MHzFT,TMS,$CDCl_3$)
1.23(3H,d,J=6.5Hz),1.59(1H,q,J-11.9Hz),2.04-2.38(2H,m), 2.60-2.94(5H,m),3.47(1H,ddd,J=1.9,4.9,17.9Hz), 3.70(3H, s),6.14(1H,dd,J=6.1,11.0Hz),7.63(1H,t,J=7.7Hz), 7.75(1H,d,J=7.8Hz),7.95(1H,d,J=1.1Hz),8.41(1H,d, J=7.8Hz),8.46 (1H,s)
MS(ESI,POS)m/z 423 $[M+H]^+$

Example 21

Synthesis of 5- (3-ethoxycarbonylpropanoyl)oxy-7-methyl-3-(3-trifluoromethylphenyl)-5,6,7,8-tetrahydro-cinnoline (compound No. 27)

Reaction was carried out in the same manner as in Example 1 except for using monoethyl succinate in place of ethoxyacetic acid, and the residue thus obtained was purified by a silica gel column chromatography (hexane/ethyl acetate = 1/1) to obtain the desired compound.
$^1$H-NMR(200MHzFT,TMS,$CDCl_3$)
1.23(3H,d,J=6.5Hz), 1.24(3H,t,J=7.2Hz), 1.59(1H,q, J=11.8Hz),2.10-2.38(2H,m),2.58-2.92(5H,m), 3.50(1H,dd, J=4.4,17.6Hz),4.15(2H,qd,J=1.0,7.2Hz), 6.15(1H,dd,J=5.9,10.9Hz),7.63(1H,t,J=7.7Hz), 7.75(1H,d,J=7.7Hz),8.00(1H, s),8.41(1H,d,J=7.7Hz), 8.47(1H,s)
MS (ESI, POS) m/z 437 $[M+H]^+$

Test Example 1

In vitro antitumor effect on mastocarcinoma cells MCF-7

By the use of RPMI 1640 medium (Asahi Tekunogurasu Co., Ltd.) containing 10% serum, 2,000 MCF-7 cells were seeded into a 96-well plate. The cells were cultured for 24 hours under conditions of 37°C and 5% $CO_2$/95% air, and then a test compound was added thereto, followed by culturing for another 3 days. Cells were stained with a 0.05% Methylene Blue solution and absorbance at 660 nM was measured with a microtiter plate reader (Benchmark Plus, mfd. by BIO RAD Laboratories Inc.). The proliferation inhibition rate was calculated by the following equation and the 50% cytostatic concentration was determined from a dose-response curve. The results obtained are shown in Table 2.

$$\text{Proliferation inhibition rate} = ((\text{absorbance for control group} - \text{absorbance for drug-treated group}) / \text{absorbance for control group}) \times 100$$

[Table 2]

| Compound No. | $IC_{50}$ value ($\mu$g/ml) |
|---|---|
| **13** | **0.135** |
| **14** | **0.148** |
| **15** | **0.171** |
| **16** | **0.166** |
| **17** | **0.139** |
| **18** | **0.981** |
| **19** | **0.908** |

(continued)

| Compound No. | $IC_{50}$ value ($\mu$g/ml) |
|---|---|
| **20** | **1.339** |
| **21** | **0.129** |
| **23** | **0.128** |
| **24** | **0.098** |
| **25** | **0.144** |
| **26** | **1.177** |
| **27** | **0.736** |
| **38** | **0.649** |
| **39** | **0.152** |
| **43** | **4.343** |

As is clear from Table 2, the compounds of the present invention suppress the proliferation of mastocarcinoma cells and hence have antitumor effect.

INDUSTRIAL APPLICABILITY

As explained above in detail, the 3-phenyltetrahydrocinnolin-5-ol derivative represented by the general formula (1), physiologically acceptable salt thereof or prodrug of either of the present invention has cytostatic activity, specifically cytostatic activity against tumor cells such as mastocarcinoma cells, and hence is effective as a cytostatic agent or an antitumor agent.

## Claims

1. A 3-phenyltetrahydrocinnolin-5-ol derivative represented by the following general formula (1):

[Formula 1]

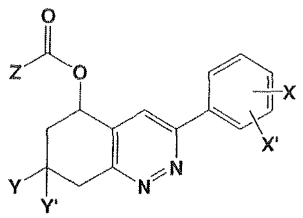

(1)

wherein Z is MO- (O is an oxygen atom), L(L')N- (N is a nitrogen atom) or A(B)CH- (C is a carbon atom and H is a hydrogen atom); M is a lower alkyl group which may have a lower alkoxy group, a lower alkylamino group or a saturated heterocyclic group as a substituent; L and L' may be taken together to represent an optionally substituted 4-to 8-membered cyclic structure group or are independently a lower alkyl group which may have a hydroxyl group, a lower alkoxy group, a carboxyl group, a lower alkoxycarbonyl group, a carbamoyl group, a lower alkylamino group or a saturated heterocyclic group as a substituent, or a hydrogen atom; A is a hydroxyl group, a lower alkyl group or a hydrogen atom; B is a lower alkyl group, a lower alkoxy group, a carboxyl group or a lower alkoxycarbonyl group, which have a substituent(s); X is a lower alkyl group, a lower alkoxycarbonyl group, a lower acylamino group, a lower alkoxy group, a trifluoromethyl group, a nitro group, a cyano group or a halogen atom; X' is a lower alkyl group, a lower alkoxycarbonyl group, a lower acylamino group, a lower alkoxy group, a trifluoromethyl group, a nitro group, a cyano group, a halogen atom or a hydrogen atom; and Y and Y' are independently a lower alkyl group or

a hydrogen atom, a physiologically acceptable salt thereof, or a prodrug of said derivative or salt.

**2.** A 3-phenyltetrahydrocinnolin-5-ol derivative, a physiologically acceptable salt thereof, or a prodrug of said derivative or salt according to claim 1, wherein Z is L(L')N- and L and L' represent an optionally substituted 4-to 8-membered cyclic structure group, which is a morpholino group, a 4-methylpiperazino group, a 4-piperidinopiperidino group or a 4-morpholinapiperidino group.

**3.** A 3-phenyltetrahydrocinnolin--5-ol derivative, a physiologically acceptable salt thereof, or a prodrug of said derivative or salt according to claim 1, wherein Z is A(B)CH-; A is a hydrogen atom; B is a (C1~C5)alkyl group substituted by one or more groups selected from a hydroxyl group, a carboxyl group, a (C1~C5) alkoxycarbonyl group, an amino group, a (C1~C5) alkylamino group, a di(C1~C5) alkylamino group, a carbamoyl group, a saturated heterocyclic group, an N-substituted carbamoyl group and a G-(C1~C5)alkoxy group (G is a carboxyl group, an N-substituted carbamoyl group or a hydrogen atom); X is a methyl group, a methoxycarbonyl group, an acetylamino group, a methoxy group, a trifluoromethyl group, a nitro group, a cyano group or a halogen atom; and X' is a trifluoromethyl group or a hydrogen atom.

**4.** A 3-phenyltetrahydrocinnolin-5-ol derivative, a physiologically acceptable salt thereof, or a prodrug of said derivative or salt according to claim 3, wherein B is a (C1~C5)alkyl group substituted by a carboxyl group, a (C1~C5)alkoxycarbonyl group, a di(C1~C5)alkylamino group or a saturated heterocyclic group; X is a trifluoromethyl group; X' is a hydrogen atom; Y is a methyl group; and Y' is a hydrogen atom.

**5.** A 3-phenyltetrahydrocinnolin-5-ol derivative, a physiologically acceptable salt thereof, or a prodrug of said derivative or salt according to claim 4, wherein B is a carboxymethyl group or a dimethylamino-2-ethyl group.

**6.** A pharmaceutical composition comprising a 3-phenyltetrahydrocinnolin-5-ol derivative, a physiologically acceptable salt thereof or a prodrug of said derivative or salt according to any one of claims 1 to 5 as an active ingredient.

**7.** A pharmaceutical composition according to claim 6, which is for inhibiting cell proliferation.

**8.** A pharmaceutical composition according to claim 6 or 7, which is for antitumor use.

**9.** Use of a 3-phenyltetrahydrocinnolin-5-ol-derivative, a physiologically acceptable salt thereof or a prodrug of said derivative or salt according to any one of claims 1 to 5 in the manufacture of a pharmaceutical composition for inhibiting cell proliferation.

**10.** Use of a 3-phenyltetrahydrocinnolin-5-ol derivative, a physiologically acceptable salt thereof or a prodrug of said derivative or salt according to any one of claims 1 to 5 in the manufacture of a pharmaceutical composition for the prophylaxis or treatment of tumors.

**11.** A method for inhibiting cell proliferation which comprises administering a 3-phenyltetrahydrocinnolin-5-ol derivative, a physiologically acceptable salt thereof or a prodrug of said derivative or salt according to any one of claims 1 to 5 to an mammal including a human being.

**12.** A method for preventing or treating tumors which comprises administering a 3-phenyltetrahydrocinnolin-5-ol derivative, a physiologically acceptable salt thereof or a prodrug of said derivative or salt according to any one of claims 1 to 5 to an mammal including a human being.

## INTERNATIONAL SEARCH REPORT

International application No.
PCT/JP2005/010494

A. CLASSIFICATION OF SUBJECT MATTER
Int.Cl[7] C07D237/26, A61K31/502, 31/5377, A61P35/00, C07D401/12

According to International Patent Classification (IPC) or to both national classification and IPC

B. FIELDS SEARCHED

Minimum documentation searched (classification system followed by classification symbols)
Int.Cl[7] C07D237/26, A61K31/502, 31/5377, A61P35/00, C07D401/12

Documentation searched other than minimum documentation to the extent that such documents are included in the fields searched

Electronic data base consulted during the international search (name of data base and, where practicable, search terms used)
REGISTRY(STN), CAPLUS(STN)

C. DOCUMENTS CONSIDERED TO BE RELEVANT

| Category* | Citation of document, with indication, where appropriate, of the relevant passages | Relevant to claim No. |
|---|---|---|
| P,X | WO 04/052866 A1 (Nippon Kayaku Co., Ltd.), 24 June, 2004 (24.06.04), Full text; particularly, page 10, line 17 to page 13; pages 57 to 59 & AU 2003289002 A1 | 1,3 |
| A | NAGARAJAN, R.K. et al., Synthesis & Reactions of 4,6,7,8-Tetrahydro-5(1H)-cinnolinones, Indian Journal of Chemistry, Section B: Organic Chemistry Including Medicinal Chemistry, 1986, Vol.25B, No.7, pages 697 to 708 | 1-10 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 11 July, 2005 (11.07.05) | 26 July, 2005 (26.07.05) |
| Name and mailing address of the ISA/ Japanese Patent Office | Authorized officer |
| Facsimile No. | Telephone No. |

Form PCT/ISA/210 (second sheet) (January 2004)

INTERNATIONAL SEARCH REPORT

International application No.
PCT/JP2005/010494

**Box No. II Observations where certain claims were found unsearchable (Continuation of item 2 of first sheet)**

This international search report has not been established in respect of certain claims under Article 17(2)(a) for the following reasons:

1. [x] Claims Nos.: 11, 12
because they relate to subject matter not required to be searched by this Authority, namely:
Claims 11 and 12 involve embodiments concerning methods for treatment of the human body by therapy and thus relate to a subject matter which this International Searching Authority is not required, under the provisions of Article 17(2)(a)(i) of the PCT (continued to extra sheet)

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.
2. [ ] As all searchable claims could be searched without effort justifying an additional fee, this Authority did not invite payment of any additional fee.
3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest**
[ ] The additional search fees were accompanied by the applicant's protest.
[ ] No protest accompanied the payment of additional search fees.

Form PCT/ISA/210 (continuation of first sheet (2)) (January 2004)

INTERNATIONAL SEARCH REPORT

International application No.
PCT/JP2005/010494

Continuation of Box No.II-1 of continuation of first sheet(2)

and Rule 39.1(iv) of the Regulations under the PCT, to search.

<With respect to statement in the claims>

The term prodrug used in claims 1-6, 9, and 10 is unclear as to what specific compounds it includes.

Form PCT/ISA/210 (extra sheet) (January 2004)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **RASHMI K.SHAH et al.** Central Nervous System Active 5-Oxo-1, 4, 5, 6, 7, 8-Hexahydrocinnolines. *Journal of Medicinal Chemistry,* 1976, vol. 19, 508-511
- **ANGELO CAROTTI et al.** Inhibition of Monoamine Oxidase-B by Condensed Pyridazines and Pyrimidines: Effects of Lipophilicity and Structure-Activity Relationships. *Journal of Medical Chemistry,* 1998, vol. 41, 3812-3820
- **K.NAGARAJAN et al.** Synthesis, Reactions of 4, 6, 7, 8-Tetrahydro-5-(1H) - cinnolinones. *Indian Journal of Chemistry,* 1986, vol. 25B, 697-708
- Development of Medicines. Molecular Design. HIROKAWA-SHOTEN Ltd, 1990, vol. 7, 163-198
- **GREEN, T.W. et al.** PROTECTIVE GROUPS IN ORGANIC SYNTHESIS. WILEY·INTERSCIENCE